# EUROPEAN PATENT APPLICATION

(11) **EP 3 210 905 A1**
(43) Date of publication of application: **30.08.2017**
(21) Application number: 15853427.1
(22) Date of filing: 20.10.2015
(51) Int. Cl.: B65D 55/06, B65D 25/20, B65D 51/24

(54) **EYE DROP CONTAINER HAVING IDENTIFYING INDICIA**

(30) Priority: 20.10.2014 JP 2014213969
(71) Applicant: Santen Pharmaceutical Co., Ltd., Higashiyodogawa-ku Osaka-shi Osaka 533-8651 (JP)
(72) Inventor: AZUMA, Yoshiyuki, Osaka-shi Osaka 530-8552 (JP)
(74) Representative: Müller-Boré & Partner Patentanwälte PartG mbB
(86) International application number: PCT/JP2015/079497
(87) International publication number: WO 2016/063845

(57) **Abstract**

Provided is an eye drop container having superior identifying power. The eye drop container includes an identifying indicium comprising a shape, a pattern, a color or combination thereof imparted to a lateral face of a container body, and a further identifying indicium identical or similar to the identifying indicium imparted to a lateral face of a cap part which is detachably attached to a dispensing mouth of the container body, the indicia making it possible to identify the unity of the container body and the cap part. The container is packaged by a sealing label that renders these identifying indicia visible when the cap part is attached to the container body.

## Description

### Technical Field

The present invention relates to an eye drop container including a container body imparted with an identifying indicium comprising a shape, a pattern, a color or combination thereof, and a cap part detachably attached to a dispensing mouth of the container body and imparted with a further identifying indicium identical or similar to the identifying indicium.

### Background Art

In general, an eye drop container is packaged by a sealing label. The functions of the sealing label lie comprise ensuring the container being an unused container not yet unsealed, and preventing unsealing thereof by an act of tampering (see e.g. Patent Document 1).

In an eye drop container disclosed in Patent Document 1, a sealing label includes an upper region for covering a cap part and a lower region for covering a container body. In the upper region, there is provided a band-like colored layer for identification along its upper end edge. And, when the sealing label is unsealed, the upper region is removed.

### Citation List

### Patent Literature

Patent Document 1: Japanese Unexamined Patent Application Publication No. 2008-068907

### Summary of Invention

### Technical Problem

In the case of the eye drop container disclosed in Patent Document 1, after unsealing, the upper region for covering the cap part is removed, so that the unity (integrity) between the container body and the cap part is lost. As a result, it may happen that a user having many eye drop containers erroneously attaches a wrong cap part different from the original cap part.

Then, in view of the above, the object of the present invention is to provide an eye drop container having superior identifying power by providing identifying indicia having unity (integrity) to the container body and to the cap part, respectively.

### Solution to Problem

According to a first aspect of the present invention, an eye drop container comprises:
an identifying indicium comprising a shape, a pattern, a color or combination thereof imparted to a lateral face of a container body;
a further identifying indicium identical or similar to the identifying indicium imparted to a lateral face of a cap part which is detachably attached to a dispensing mouth of the container body, thus allowing identification of unity between the container body and the cap part; and
the container being packaged by a sealing label that renders the identifying indicium and the further identifying indicium visible when the cap part is attached to the container body.

Namely, according to the first aspect invention above, identifying indicia are imparted to the lateral face of the container body and the lateral face of the cap part and the container body and the cap part are sealed by a transparent or semi-transparent sealing label having no identifying indicium.

In this way, in the first aspect invention, in order to ensure the container being an unused container not yet unsealed and also to prevent unsealing by a tampering act, it is provided that this container is packaged by a transparent or semi-transparent sealing label. However, in view of the fact that identifying indicia are provided in advance to the lateral face of the container body and the lateral face of the cap part without packaging by the sealing label, respectively, even an eye drop container un-packaged by the sealing label intentionally will retain the unity between the container body and the cap part. Thus, such container too is understood be encompassed within the scope of the first aspect invention.

According to a second aspect of the present invention, an eye drop container comprises:
an identifying indicium comprising a shape, a pattern, a color or combination thereof imparted to a lateral face of a container body; and
the container being packaged by a sealing label in such a manner as to render the identifying indicium visible when a cap part that is detachably attached to a dispensing mouth of the container body is attached to the container body;
wherein a further identifying indicium identical or similar to the identifying indicium is provided in the sealing label in either an upper region for covering the cap part wholly or partially or a region comprising the upper region and a cutaway region disposed between the upper region and a lower region for covering the container body wholly or partially, thus allowing identification of unity between the container body and the cap part;
wherein in a border between the cutaway region and the upper region and in a border between the cutaway region and the lower region, a cutaway line is provided respectively; and
wherein the upper region remains attached to the cap part after unsealing of the sealing label, thus allowing identification of unity between the container body and the cap portion.

Namely, in the second aspect invention, an identifying indicium is provided in a lateral face of the container body and also this container body is packaged by a transparent or semi-transparent sealing label having no identifying indicium whereas the cap part is packaged by a sealing label having an identifying indicium imparted thereto (see Second Embodiment to be described later).

According to a third aspect of the present invention, an eye drop container comprises:
an identifying indicium comprising a shape, a pattern, a color or combination thereof imparted to a lateral face of a cap part detachably attached to a dispensing mouth of a container body; and
the container being packaged by a sealing label that renders the identifying indicium visible when the cap part is attached to the container body;
wherein a further identifying indicium identical or similar to the identifying indicium is provided in the sealing label in a lower region thereof configured to cover the container body wholly or partially, thus allowing identification of unity between the container body and the cap part;
wherein in a border between an upper region of the sealing label configured to cover the cap part wholly or partially and the lower region, a cutaway line is provided; and
wherein the lower region remains attached to the cap part after unsealing of the sealing label, thus allowing identification of unity between the container body and the cap portion.

Namely, in the third aspect invention, the container body is packaged by a sealing label having an identifying indicium imparted thereto and a further identifying indicium is imparted to a lateral face of the cap part, and also the cap part is packaged by a transparent or semi-transparent sealing label having no identifying indicium imparted thereto (see Third Embodiment to described later).

According to the first through third aspect inventions described above, a further identifying indicium identical or similar to an identifying indicium comprising a shape, a pattern, a color or combination thereof imparted to a lateral face of the container body or a lower region of the sealing label is imparted to e.g. a lateral face of the cap part or an upper region of the sealing label, whereby the unity between the container body and the cap part can be easily identified. Further, the identifying indicium comprising a shape, a pattern, a color or combination thereof can be easily designated by a user and can take a great variety of forms such as graphical shapes such as a triangle, a square, a rhombus, a circle, a star-shape, a crescent shape, a heart-shape, etc. or a single character such as an alphabet or a Japanese *`hiragana'* character, etc., which will unlikely cause erroneous identification among a plurality of users, so even when there exist many kinds of contents for the eye drop container, an identifying indicium to be commonly applied to each kind of contents can be readily created. Further, the identifying indicium comprising a shape, a pattern, a color or combination thereof is not particularly limited in its color in each presentation thereof, but it is preferred that the indicium be provided in a same single color or two colors. The identifying indicium comprising a shape, a pattern, a color or combination thereof can be directly printed on the lateral face of the container body or the lateral face or a top face of the cap part, or a paper sheet or film printed with such identifying indicium can be applied to the latera face of the container body or the lateral face or a top face of the cap part. Further alternatively, if a treatment for providing unevenness or three-dimensional coloring at the time of forming of the eye drop container or an embossing treatment in the sealing label, etc. is provided, such will render identification easy for visually handicapped people also.

Further, after unsealing too, these identifying indicia remain attached to the lateral faces of the container body and the cap part, so that the unity between the container body and the cap part is maintained throughout the use period of the eye drop container.

Incidentally, the identifying indicium or the further identifying indicium identical or similar to the identifying indicium can be provided in a plurality to the lateral face of the cap part, the lateral face of the container body or the sealing label. In this case, the identifying indicia can be visually identified from any angle in the circumference direction of the eye drop container.

In the eye drop container of the present invention, in order to further enhance the unity (integrity) between the container body and the cap part, a further identifying indicium identical or similar to the identifying indicium can be imparted to a top face of the cap part.

With provision of an identifying indicium identical or similar to the identifying indicium imparted to the lateral face of the container body or the lateral face of the cap part provided to the top face of the cap part, it becomes possible to identify the unity (integrity) between the container body and the cap part from the upper side of the eye drop container also.

### Brief Description of Drawings

[Fig.1] is a perspective view showing identifying indicia imparted to a container body and a cap part relating to First Embodiment,
[Fig. 2] is a front view showing a sealing label relating to First Embodiment,
[Fig. 3] is a front view showing the sealing label relating to First Embodiment,
[Fig. 4] is a perspective view showing an eye drop container packaged by the sealing label relating to First Embodiment,
[Fig. 5] is a perspective view showing the eye drop container relating to First Embodiment with the sealing label being unsealed,
[Fig. 6] is a perspective view showing the eye drop container relating to First Embodiment, with an identifying indicium being imparted thereto,
[Fig.7] is a perspective view showing an identifying indicium being imparted to a container body relating to Second Embodiment,
[Fig 8] is a front view showing an identifying indicium imparted to the sealing label relating to Second Embodiment,
[Fig. 9] is a perspective view showing an eye drop container packaged by the sealing label relating to Second Embodiment,
[Fig.10] is a perspective view showing the eye drop container relating to Second Embodiment, with the sealing label being unsealed,
[Fig.11] is a front view showing an identifying indicium imparted to the sealing label relating to Second Embodiment,
[Fig. 12] is a perspective view showing an identifying indicium imparted to a cap part relating to Third Embodiment,
[Fig.13] is a front view showing an identifying indicium imparted to the sealing label relating to Third Embodiment,
[Fig.14] is a front view showing an identifying indicium imparted to the sealing label relating to Third Embodiment,
[Fig. 15] is a perspective view showing an eye drop container relating to Third Embodiment packaged by the sealing label,
[Fig.16] is a perspective view showing the eye drop container relating to Third Embodiment, with the sealing label being unsealed,
[Fig.17] is a front view showing a sealing label relating to a further embodiment,
[Fig. 18] is a perspective view showing the eye drop container relating to First Embodiment imparted with an identifying indicium,
[Fig. 19] is a perspective view showing the eye drop container relating to First Embodiment imparted with an identifying indicium,
[Fig. 20] is a perspective view showing the eye drop container relating to First Embodiment imparted with an identifying indicium,
[Fig. 21] is a perspective view showing the eye drop container relating to First Embodiment imparted with an identifying indicium, and
[Fig. 22] is a perspective view showing the eye drop container relating to First Embodiment imparted with an identifying indicium.

### Description of Embodiments

Next, embodiments of an eye drop container relating to the present invention will be explained with reference to the drawings. In this embodiment, there will be explained a case wherein as an example of an eye drop container 2, the eye drop container 2 is packaged by a sealing label 1. It is understood however that the present invention is not limited to the following embodiments, but many variations are possible.

### [First Embodiment]

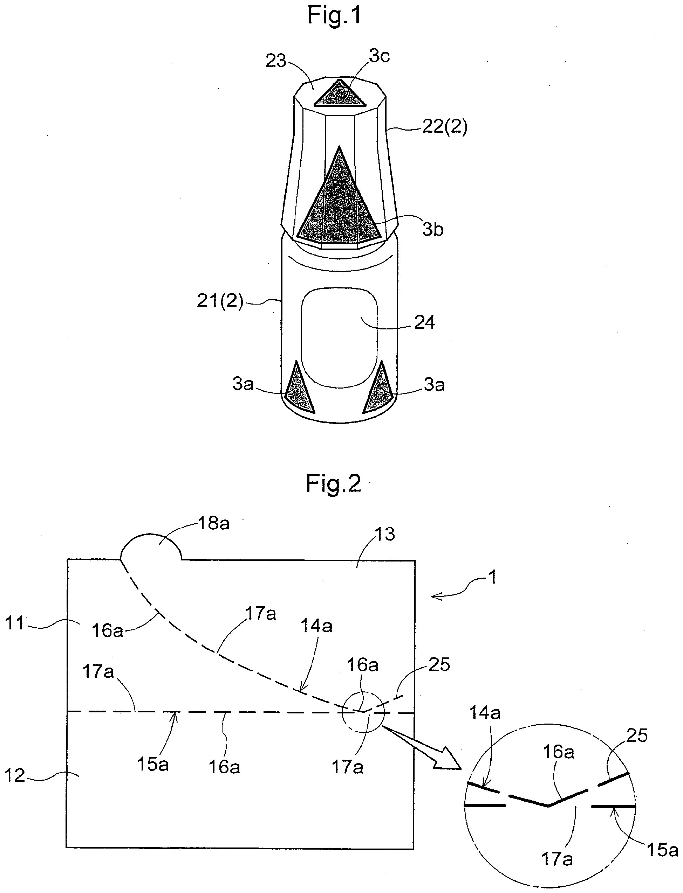

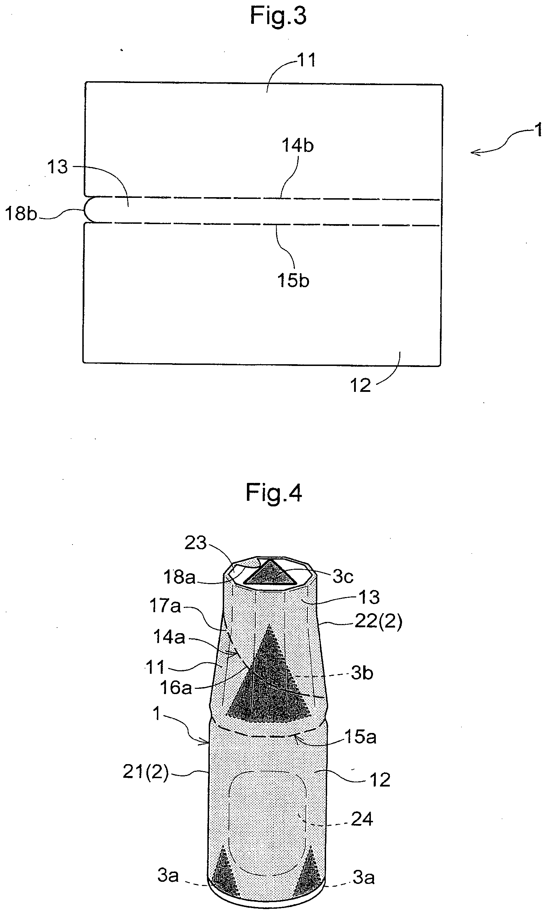

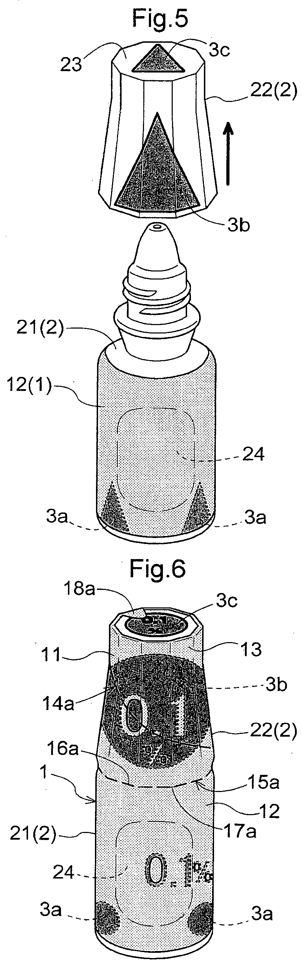

Fig.1 shows a perspective view in which identifying indicia 3a, 3b (a shape, a pattern, a color or combination thereof) are imparted to a lateral face of a container body 21 and a lateral face of a cap part 22 relating to First Embodiment. Fig. 2 and Fig. 3 show plan views of a sealing label 1 relating to First Embodiment. Fig. 4 and Fig. 5 show perspective views of an eye drop container 2 under a state where the sealing label 1 is wrapped around the eye drop container 2 and under a state where the sealing label 1 is unsealed.

The eye drop container 2 includes the container body 21 with an elongate cylindrical shape having a depressing recess 24 at the center thereof and the truncated conical shaped cap part 22 having a polygonal external face. To the lateral face of the container body 21 and the lateral face of the cap part 22, one or more (two in the example illustrated) of identifying indicium (or indicia) 3a, 3b having an identical or similar triangular shape is/are imparted. Here, in Fig. 1, only the identifying indicium 3b provided in the front face of the cap part 22 is visible, but in fact, another identifying indicium 3b is imparted to the back face of the cap part 22 also.

A method of imparting the identifying indicia 3a, 3b to the lateral face of the container body 21 and the lateral face of the cap part 22 can be application of coloring to the eye drop container 2 after formation thereof or using of resins of different colors at the time of molding such as two-color molding. Further, the method of imparting the identifying indicia 3a, 3b to the container body 21 and the cap part 22 can also be affixing of paper sheet or film printed with the identifying indicia 3a, 3b to the lateral face of the container body 21 or the lateral face of the cap part 22. In this way, the method is not particularly limited. Incidentally, the identifying indicia 3a, 3b can be provided with unevenness surface treatment or three-dimensional coloring also.

The sealing label 1 used in this embodiment, as shown in Fig. 4, is wrapped around the eye drop container 2 in such a manner to render the identifying indicia 3a, 3b visible when the cap part 22 is attached to the container body 21.

In order to render the identifying indicia 3a, 3b visible, it is preferred that the sealing label 1 be made transparent or semi-transparent or the sealing label 1 be not applied to the portions of the identifying indicia 3a, 3b, etc.

This sealing label 1 comprises a resin film having a rectangular shape as shown in Fig. 2 and Fig. 3, for example a shrink label which can be wrapped and retained around the eye drop container 2 by heat-shrunk. Incidentally, the sealing label 1 is not limited to such shrink label, but can also be a stretch label, etc.

Further, the sealing label 1 can be affixed by firstly being wrapped around the container body 21 and the cap part 22 and then heat-shrunk. Or, the sealing label 1 can be prepared in a cylindrical shape first and wrapped around the container body 21 and the cap part 22 first, then being heat-shrunk thereon.

As shown in Fig. 2, the sealing label 1 includes an upper region 11 for covering the cap part 22 wholly or partially, and a lower region 12 for covering the container body 21 wholly or partially. The upper region 11 used in this embodiment is configured to cover the lateral face of the cap part 22 and a portion of the top face 23 thereof, whereas the lower region 12 is configured to cover the lateral face of the container body 21. Incidentally, the covering extents of the upper region 11 and the lower region 12 are not particularly limited, as long as they respectively cover the cap part 22 or the container body 21 either wholly or partially.

In a border between the upper region 11 and the lower region 12, there is provided a horizontal perforation line 15a (a 'cutaway line'). Further, in the upper region 11, there is provided an inclined perforation line 14a which extends obliquely from an unsealing pinching portion 18a formed at the upper side thereof to the horizontal perforation line 15a. A protrusion amount of the pinching portion 18a is set to an appropriate length according to heat-shrinkage characteristics of the sealing label 1, so as to effectively resist 'twisting' deformation thereof. Incidentally, the shape of the leading end of the pinching portion 18a is a semi-circular arc, but it can be appropriately set to other shapes such an angular shape, a triangular shape, etc. also in consideration to such factors as shrinkage strength, readiness of pinching, etc. Further, the position of the pinching portion 18a is not limited to the upper side of the upper region 11, but can be set to the right side or left side, etc. thereof.

Here, in the context of this disclosure, the language 'perforation line' means a group of through holes, similar to sewing stitches, comprising a plurality of through holes (cut portions 16a) extending through the front side and the back side of the sealing label 1 and defined with predetermined spacing therebetween. In such perforation line, there exist non-through portions (uncut portions 17a) between adjacent through holes. In this, it is preferred that the cut portions 16a are formed longer than the uncut portions 17a. This arrangement provides much more smooth breaking of the 'perforation line'. Incidentally, the frontal shape of one cut portion 16a is not particularly limited, but can be a narrow elongate straight shape, a needle hole shape (punched out, approximately circular or elliptical shape) as long as it allows smooth breaking. Fig 2 shows a narrow elongate linear shaped one.

In the sealing label 1 according to this embodiment, for the inclined perforation line 14a, its contact area with the horizontal perforation line 15a is constituted of a V-shaped cut portion 16a and there is provided a relatively short additional perforation line 25 in left-right symmetry with respect to the inclined perforation line 14a. Further, in the horizontal perforation line 15a, its area in contact with the inclined perforation line 14a is provided as a relatively large uncut portion 17a. Incidentally, the additional perforation line 25 can be omitted and the relatively large uncut portion 17a need not be provided in the contact area between the inclined perforation line 14a and the horizontal perforation line 15a.

Further, the sealing label 1 in this embodiment can be configured as shown in Fig. 3. This sealing label 1 includes an upper region 11 for covering the cap part 22 wholly or partially, a lower region 12 for covering the container body 21 wholly or partially and a cutaway region 13 positioned between the upper region 11 and the lower region 12, when the cap part 22 is attached to the container body 21. The upper region 11 covers the lateral face of the cap part 22 and a portion of its top face 23. The lower region 12 covers the lateral face of the container body 21. Incidentally, the covering extensions provided by the upper region 11 and the lower region 12 are not particularly limited as long as they respectively cover the cap part 22 and the container body 21 wholly or partially.

In a border between the cutaway region 13 and the upper region 11, a perforation line 14b (a 'cutaway line') is provided. In a border between the cutaway region 13 and the lower region 12, a perforation line 15b (a 'cutaway line') is provided. Namely, the cutaway region 13 has a horizontally elongate band-like shape and extends from one end to the other end thereof along the direction of wrapping the sealing label 1 around the eye drop container 2. And, in the vertical direction of the cutaway region 13, the rectangular-shaped upper region 11 and lower region 12 are formed via the perforation lines 14b, 15b.

Further, in the cutaway region 13, there is provided an unsealing pinching portion 18b formed at the end thereof. A protrusion amount of this pinching portion 18b is set to an appropriate length according to heat-shrinkage characteristics of the sealing label 1, so as to effectively resist 'twisting' deformation thereof.

Fig. 4 and Fig. 5 show an example in which the sealing label 1 shown in Fig. 2 is wrapped around the eye drop container 2. This eye drop container 2 is used after unsealing the sealing label 1 by an operation of breaking the cutaway region 13 obliquely (right-downward direction) of the eye drop container 2 with gripping the pinching portion 18a or by an operation of rotating the cap part 22.

In the case of unsealing the sealing label 1 with the operation of rotating the cap part 22, in the contact area between the inclined perforation line 14a and the horizontal perforation line 15a, the V-shaped cut portion 16a will be cut prior to the uncut portion 17a of the horizontal perforation line 15a, and then the inclined perforation line 14a and the horizontal perforation line 15a will be cut one after another. With this, there will remain a trace of unsealing in an unrecoverable manner. So, tampering act can be effectively prevented. Finally, at least the upper region 11 will be removed from the cap part 22.

As shown in Fig. 5, once the sealing label 1 of the eye drop container 2 has been unsealed, at least the upper region 11 is removed, but the identifying indicia 3a, 3b remain attached to the lateral face of the container body 21 and the lateral face of the cap part 22. Therefore, with reference to these respective identifying indicia 3a, 3b, the unity (integrity) between the container body 21 and the cap part 22 can be easily identified. Incidentally, in the case of the sealing label 1 shown in Fig. 3 being wrapped around the eye drop container 2 too, the identifying indicia 3a, 3b will remain attached to the lateral face of the container body 21 and the lateral face of the cap part 22. Therefore, the unity (integrity) between the container body 21 and the cap part 22 can be easily identified.

Next, various modes the identifying indicia 3a, 3b respectively comprising a shape, a pattern, a color or combination thereof and attached to the lateral face of the container body 21 and the lateral face of the cap part 22 will be explained. The identifying indicia 3a, 3b respectively comprise a shape, a pattern, a color or combination thereof. Preferably, the identifying indicia 3a, 3b will be fabricated in consideration to e.g. medical components contained in the eye drop container 2 or amounts thereof, etc.

As shown in Fig. 1 and Fig. 4, in the lateral face of the container body 21 and the lateral face of the cap part 22, one or more identifying indicia having identical or similar triangular shape is/are imparted as the identifying indicia 3a, 3b. The identifying indicia 3a, 3b employed in this embodiment have a color added to their triangular shapes.

By varying the shape, pattern, color of the identifying indicia 3a, 3b, many variations can be created. So that, even when many various kinds of contents are housed in the eye drop container 2, it is readily possible to create identifying indicia 3a, 3b to be commonly imparted to each kind of contents.

As shown in Fig. 5, even when the sealing label 1 is unsealed in the eye drop container 2, the identifying indicia 3a, 3b remain imparted to the lateral face of the container body 21 and the lateral face of the cap part 22. So, the identifying power of these identifying indicia 3a, 3b can be maintained. Therefore, during a period of use of the eye drop container 2, the unity between the container body 21 and the cap part 22 is maintained, so that by visually identifying the identifying indicium 3a of the container body 21 and the identifying indicium 3b of the cap part 22, a user can attach the cap part 22 to the container body 21 without an error. Further, if an unevenness treatment or three-dimensional coloring is added to the identifying indicia 3a, 3b, identification will be readily possible by a visually handicapped person also.

The two identifying indicia 3b imparted to the lateral face of the cap part 22 are located in the front face and the back face of this cap part 22, respectively. Further, in the lateral face of the container body 21, two identifying indicia 3a are provided in left-right symmetry relative to the center of the identifying indicium 3a located in the front face of the cap part 22. Namely, the two identifying indicia 3a of the container body 21 are disposed on the opposed lateral sides as seen from the front face of the container body 21, as shown in Fig. 1. Incidentally, the number of the identifying indicia 3a, 3b imparted to the container body 21 or the cap part 22 is not limited to one or two, but can be three or more.

If a plurality of identifying indicia 3a, 3b are imparted to the lateral face of the container body 21 and the lateral face of the cap part 22, this will make it possible for the identifying indicia 3a, 3b to be identified from any direction, so that erroneous attaching of the cap part 22 to the container body 21 will be prevented in a reliable manner.

Further, in the lower region 12 of the sealing label 1 which is not to be removed at the time of unsealing, a rectangular-shaped character displaying portion in e.g. white background can be provided. And, preferably, in this character displaying portion, medical components, serial manufacturing number of the eye drop medicine, etc. will be shown.

As shown in Fig. 1, in the top face 23 of the cap part 22 too, an identifying indicium 3c identical or similar to the identifying indicia 3a, 3b can be provided. This identifying indicium 3c to be provided in the top face 23 can be formed integrally with the cap part 22 or can be affixed as a sheet of paper or a film printed separately with the identifying indicium 3c to the top face 23 of the cap part 22. With this provision of the identifying indicium 3c in the top face 23, the unity between the container body 21 and the cap part 22 can be identified from the upper side of the cap part 22 also. Incidentally, a method for imparting the identifying indicium 3c to the top face 23 can vary in many ways, just like the container body 21 and the cap part 22 described above. Further, this identifying indicium 3c in the top face 23 can be omitted.

Fig. 6 shows different modes of the identifying indicia 3a, 3b, 3c to be imparted to the lateral face of the container body 21, the lateral face of the cap part 22 and the top face 23 of the cap part 22.

To the lateral face of the cap part 22, the lateral face of the container body 21 and the top face 23 of the cap part 22, there are imparted one or plurality of identical or similar circular shapes as the identifying indicia 3a, 3b, 3c. Further, within the identifying indicia 3b, 3c present in the lateral face and the top face 23 of the cap part 22, medical component contents of the eye drop container 2 are shown. Further, at a position adjacent the front face center of the lateral face of the container body 21, medical components contents same as those described in the identifying indicia 3b, 3c present in the lateral face and the top face 23 of the cap part 22 are shown.

Incidentally, as an example of medical component content, a numeral (0.1%) is shown for meaning that 1 mg of medical agent is contained relative to 1 ml of eye drop solution. In this way, a value according to medical agent content can be shown as needed. Further, in the above, there was disclosed the case in which two identical or similar circles are provided as the identifying indicia 3a, 3b imparted to the lateral face of the container body 21 and the lateral face of the cap part 22. However, the kind and the number of the identifying indicia 3a, 3b are not particularly limited. Further, a medical agent content showing can be provided within the identifying indicia 3a present in the lateral face of the container body 21 or such medical agent content showing can be provided in the lower region 12 of the sealing label 1 also. Moreover, such medical agent content showing can be provided in one of the two identifying indicia 3b provided in the lateral face of the cap part 22. Further alternatively, the identifying indicium 3c in the top face 23 and/or the medical agent content can be omitted at all.

In this way, even when e.g. the identifying indicia 3a, 3b, 3c of an identical shape are imparted and there exist two kinds of eye drop containers 2 having different medical agent contents from each other, since a common medical agent content is shown in the lateral face of the container body 21 and the lateral face and the top face 23 of the cap part 22, such inconvenience as erroneous attachment of the cap part 22 to the container body 21 can be prevented in a reliable manner.

### [Second Embodiment]

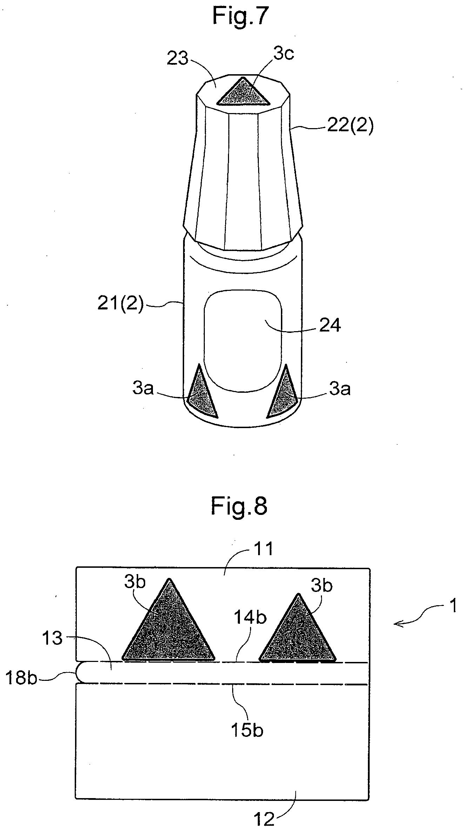

With reference to Figs. 7-11, there will be explained mainly arrangements different from First Embodiment. For assisting understanding of the illustrations, in the following discussion, same reference marks/numerals as those of First Embodiment will be used. But, the invention is not limited thereto in particular.

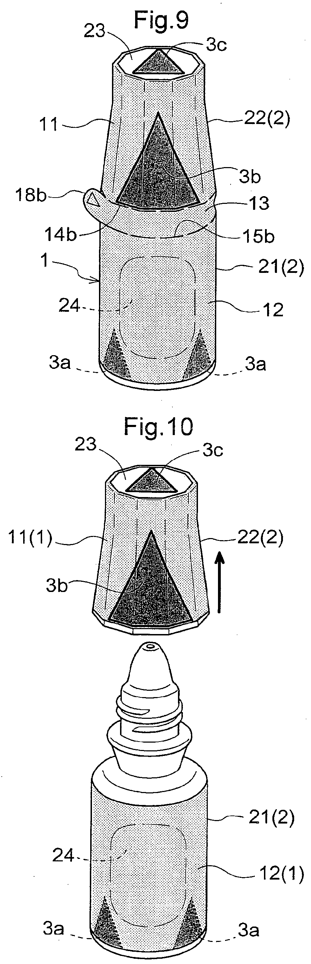

Fig 7 shows a perspective view showing a configuration in which identifying indicia 3a (a shape, a pattern, a color or combination thereof) imparted to the lateral face of the container body 21 relating to Second Embodiment. Fig. 8 shows a plane view of the sealing label 1 relating to Second Embodiment. Fig. 9 and Fig. 10 show perspective views of the eye drop container 2 under a condition of the eye drop container 2 is wrapped by the sealing label 1 relating to this embodiment and under a condition of the sealing label 1 being unsealed.

The eye drop container 2 includes a container body 21 and a cap part 22 and in the lateral face of the container body 21,one or more triangular shapes (two of them in the illustration) is/ are imparted as the identifying indicia 3a comprising a shape, a pattern, a color or combination thereof. Incidentally, the shape of the eye drop container 2 and the method of imparting the identifying indicia 3a to the lateral face of the container body 21 are identical to those of First Embodiment.

The sealing label 1 in this embodiment is wrapped around the eye drop container 2 in such a manner as to allow visual identification of the identifying indicia 3a imparted to the lateral face of the container body 21 when the cap part 22 is attached to the container body 21, as shown in Fig. 9. For allowing the visual identification of the identifying indicia 3b, it is preferred that the sealing label 1 be formed transparent or semi-transparent or that the sealing label 1 not cover the portion of the identifying indicia 3a. Incidentally, the mode of the sealing label 1 in this embodiment is same as the sealing label 1 of First Embodiment show in Fig. 3 except that the identifying indicium 3b is provided in the upper region 11.

As shown in Fig. 8 and Fig. 9, in the upper region 11 of the sealing label 1, one or a plurality (two of them in the illustrated example) of triangular shapes (identifying indicia 3b) identical or similar to the identifying indicium 3a imparted to the lateral face of the container body 21 are provided. The identifying indicia 3a, 3b of this embodiment have coloring added to their triangular shape.

More particularly, in the upper region 11 of the sealing label 1, two identifying indicia 3b are provided in its left-right direction. Preferably, these two identifying indicia 3b of the upper region 11 will be disposed such that the respective identifying indicia 3a, 3b are disposed in the front face and the back face of the cap part 22 when the eye drop container 2 is wrapped by the sealing label 1. Incidentally, the number of the identifying indicia 3a, 3b imparted to the lateral face of the container body 21 or the upper region 11 is not limited to two, but can be three or more.

Provision of the plurality of identifying indicia 3a, 3b to the lateral face of the container body 21 and the upper region 11 allows identification of the identifying indicia 3a, 3b from any direction, so that erroneous attachment of the cap part 22 to the container body 21 can be prevented in a reliable manner. Further, like First Embodiment, it is preferred that the identifying indicium 3c be provided in the top face 23 of the cap part 22 also.

The eye drop container 2, as shown in Fig. 9 and Fig. 10, will be ready for use after unsealing the sealing label 1 by an operation of breaking a cutaway region 13 along the circumferential direction of the eye drop container 2 with pinching the pinching portion 18b. Incidentally, the leading end shape of the pinching portion 18b is semi-circular in this embodiment. However, it can be decided appropriately as an angular shape, a triangular shape, etc., in consideration to such factors as shrinkage resistance, readiness of pinching, etc. Further, the position of the pinching portion 18b is not limited to the position at the end of the cutaway region 13. Further, the pinching portion 18b can be caused to project from the left side of the upper region 11 or the lower region 12.

For the sealing label 1, it is preferred that an adhesive agent be applied at least to a predetermined area in the back face of the upper region 11. As shown in Fig. 10, when the sealing label 1 of the eye drop container 2 is unsealed, the cutaway region 13 will be removed, but at least the upper region 11 will remain attached to the cap part 22.

When the eye drop container 2 is unsealed with gripping the pinching portion 18b of the sealing label 1, at least the upper region 11 remains attached to the cap part 22. Therefore, with the identifying indicia 3a of the container body 21, the identifying power of the eye drop container 2 is maintained.

Therefore, during the period of use of the eye drop container 2, the unity between the container body 21 and the cap part 22 is maintained. So, as a user visually identifies the identifying indicium 3a of the container body 21 and the identifying indicium 3b of the cap part 22, the user can attach the cap part 22 to the container body 21 without an error. Further, if e.g. an unevenness providing treatment or three-dimensional coloring is added at the time of forming of the container body 21 or an emboss treatment is added to the sealing label 1, then, identification by a visually handicapped person too can be made easily.

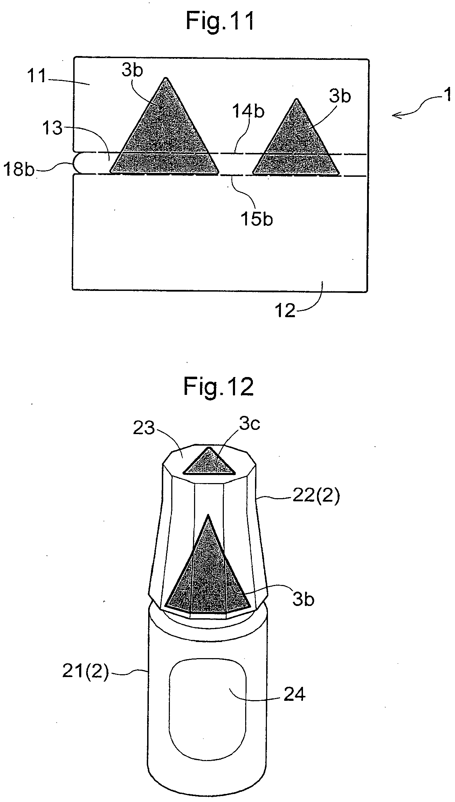

Fig.11 shows a different mode of the identifying indicium 3b to be imparted to the front face including the region of at least the upper region 11. The identifying indicium 3b in this embodiment is imparted to the region consisting of the upper region 11 and the cutaway region 13.

Incidentally, aside from such modes as above, a plurality of identifying indicia 3b can be appropriately imparted to any combination of the upper region 11 and a region consisting of the upper region 11 and the cutaway region 13.

In the above mode of the identifying indicium 3b being imparted to the cutaway region 13 also, since this identifying indicium 3b is formed in the upper region 11 in such a manner to allow imaging of its original shape, the identifying power is not lost even after the cutaway region 13 is broken. Further, absence of the portion of the cutaway region 13 in the identifying indicium 3b allows judgment of presence/absence of unsealing of the sealing label 1.

### [Third Embodiment]

With reference to Figs. 12 through 16, arrangements different from First Embodiment and Second Embodiment will mainly be explained. Incidentally, for assisting understanding of the illustrations, in the following discussion, same reference marks/numerals as those of First Embodiment and Second Embodiment will be used. But, the invention is not limited thereto in particular.

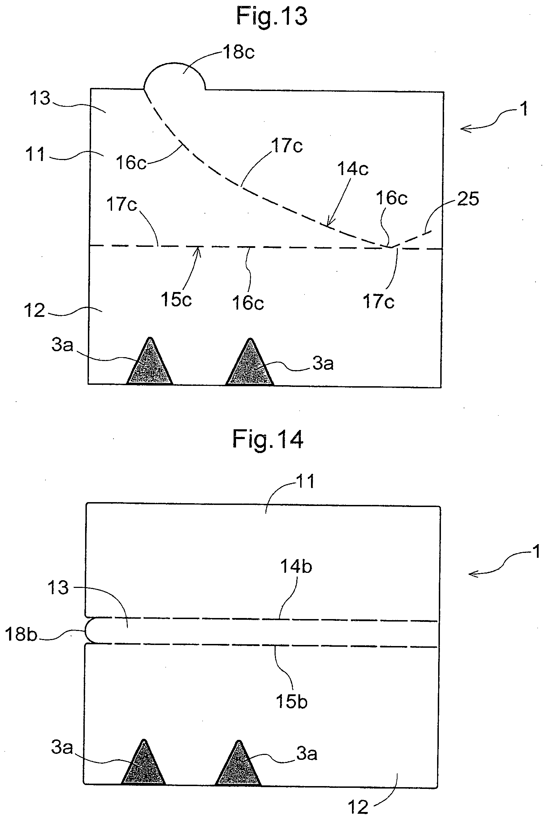

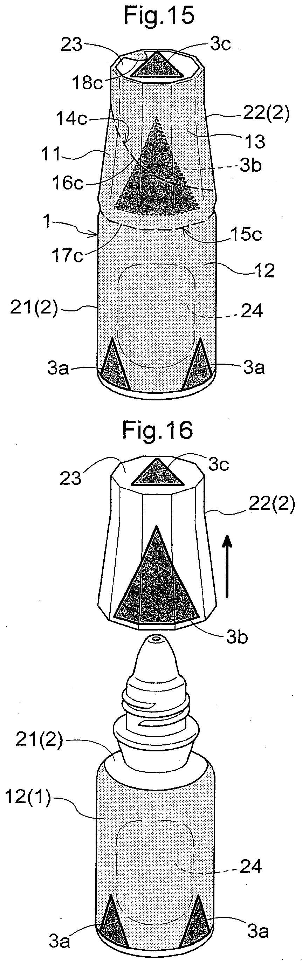

Fig. 12 shows a perspective view showing a configuration in which an identifying indicium 3b (a shape, a pattern, a color or combination thereof) is imparted to the lateral face of the cap part 22 relating to Third Embodiment. Fig. 13 shows a plane view of the sealing label 1 relating to Third Embodiment. Fig. 15 and Fig. 16 show perspective views of the eye drop container 2 under a condition of the eye drop container 2 is wrapped by the sealing label 1 relating to this embodiment and under a condition of the sealing label 1 being unsealed.

The eye drop container 2 includes a container body 21 and a cap part 22, and in the lateral face of the cap part 22, one or more triangular shapes (two of them in the illustration) is/ are imparted as the identifying indicia 3b comprising a shape, a pattern, a color or combination thereof. Incidentally, the shape of the eye drop container 2 and the method of imparting the identifying indicia 3b to the lateral face of the cap part 22 are identical to those of First Embodiment.

The sealing label 1 in this embodiment is wrapped around the eye drop container 2 in such a manner as to allow visual identification of the identifying indicia 3b when the cap part 22 is attached to the container body 21, as shown in Fig. 15. For allowing the visual identification of the identifying indicia 3b, it is preferred that the sealing label 1 be formed transparent or semi-transparent or that the sealing label 1 not cover the portion of the identifying indicia 3b. Incidentally, the mode of the sealing label 1 in this embodiment is same as the sealing label 1 of First Embodiment show in Fig. 2 and Fig. 3 except that the identifying indicium 3b is provided in the lower region 12, as shown in Fig. 13 and Fig. 14.

As shown in Fig. 13 and Fig. 14, in the lower region 12 of the sealing label 1, two identifying indicia 3a are provided in right-left symmetry as seen from the front face of the container body 21, when the sealing label 1 is wrapped around the eye drop container 2. Incidentally, the number of the identifying indicia 3a, 3b imparted to the lower region 12 or the lateral face of the cap part 22 is not limited to two, but can be three or more. Provision of the plurality of identifying indicia 3a, 3b to the lower region 12 and the lateral face of the cap part 22 allows identification of the identifying indicia 3a, 3b from any direction, so that erroneous attachment of the cap part 22 to the container body 21 can be prevented in a reliable manner.

Fig. 15 and Fig. 16 show an example in which the sealing label 1 shown in Fig. 13 is wrapped around the eye drop container 2. The eye drop container 2 will be ready for use after unsealing the sealing label 1 by an operation of breaking a cutaway region 13 along the oblique direction (right downward direction) of the eye drop container 2 with pinching of the pinching portion 18a or an operation of rotating the cap part 22.

For the sealing label 1 relating to this embodiment, it is preferred that an adhesive agent be applied to a predetermined area in the back face of the lower region 12. As shown in Fig. 16, when the sealing label 1 of the eye drop container 2 is unsealed, the upper region 11 and the cutaway region 13 will be removed, but at least the lower region 12 imparted with the identifying indicium 3a will remain attached to the container body 21. Therefore, with reference to these respective identifying indicia 3a, 3b imparted to the lower region 12 and the lateral face of the cap part 22, the unity (integrity) between the container body 21 and the cap part 22 can be identified easily. Further, if e.g. an unevenness providing treatment or three-dimensional coloring is added at the time of forming of the cap part 22 or an emboss treatment is added to the sealing label 1, then, identification by a visually handicapped person too can be made easily. Incidentally, like First Embodiment, it is preferred that the top face 23 of the cap part 22 be provided also with the identifying indicium 3c identical or similar to the identifying indicia 3a, 3b.

### [Other Embodiments]

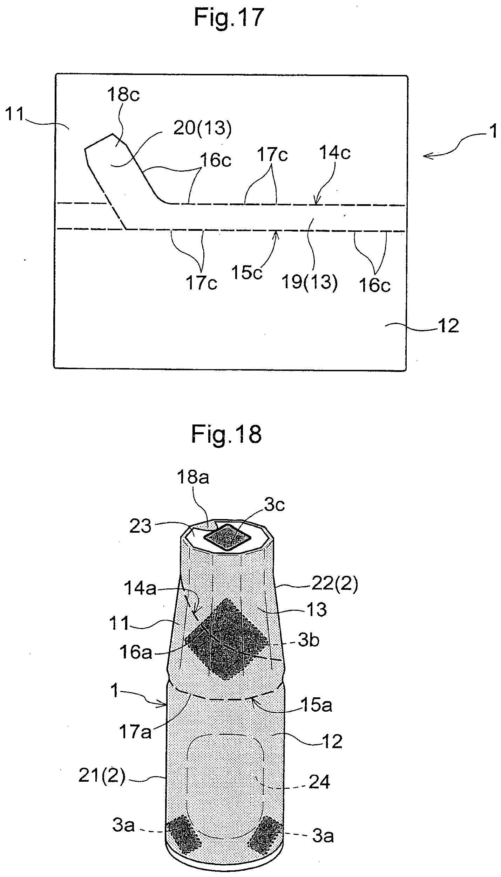

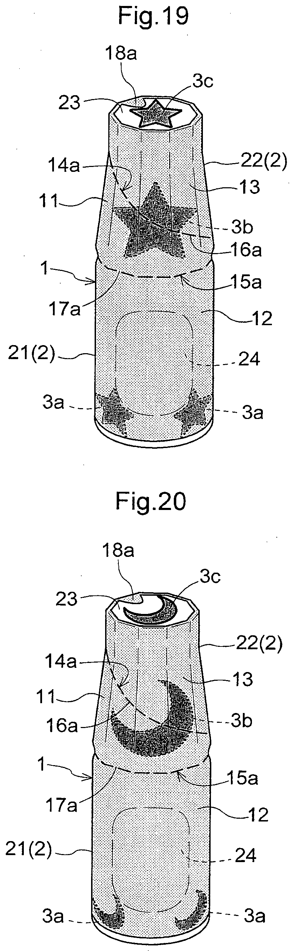

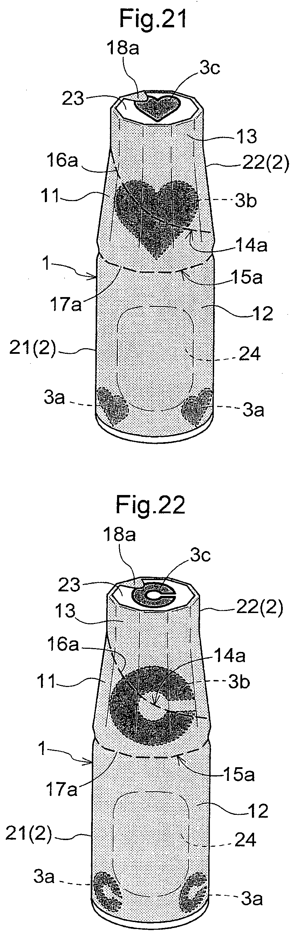

(1) The sealing label 1 relating to the above-described embodiment can be configured as shown in Fig. 17. This sealing label 1 includes an upper region 11 for covering the cap part 22 wholly or partially, a lower region 12 for covering the container body 21 wholly or partially and a cutaway region 13 positioned between the upper region 11 and the lower region 12, when the cap part 22 is attached to the container body 21. In the cutaway region 13, there are formed a first region 19 that extends from one end to the other end along the direction of wrapping the sealing label 1 around the eye drop container 2 and a second region 20 that extends in the direction from the first region 19 to the upper region 11. As this second region 20 is inclined, the pinching portion 18c can be easily pinched at the time of unsealing of the sealing label 1. Incidentally, the second region 20 can be formed without inclination, but formed perpendicular to the first region 19. The second region 20 includes a right side on which the cut portions 16c are connected continuously to the perforation line 14c and a left side on which the cut portions 16c and the uncut portions 17c are connected continuously with the perforation line 15c. In this arrangement, for readiness of pinching of the pinching portion 18c, the cut portions 16c on the right side are formed large and the uncut portions 17c are provided in the left side. Incidentally, uncut portions 17c can be provided on the right side also.
(2) The identifying indicia 3a, 3b, 3c imparted to the container body 21, the cap part 22 and the sealing label 1 in this embodiment can have e.g. a rhombus shape as shown in Fig.18, a star-shape as shown in Fig. 19, a moon-shape as shown in Fig. 20, a heart-shape as shown in Fig. 21, a C-shape as shown in Fig. 22. These graphic shapes are only non-limiting examples. With combination of a shape, a pattern, a color, various identifying indicia 3a, 3b, 3c can be imparted to the container body 21, the cap part 22 and the sealing label 1.
(3) The shape of the cutaway line in the sealing label 1 in the above embodiments can be any shape. Also, in First Embodiment, with omission of the cutaway line from the label 1, the sealing label 1 can be configured to be stripped off the eye drop container 2. In this case too, the identifying indicia 3a, 3b are imparted to the lateral face of the container body 21 and the lateral face of the cap part 22, so that the unity (integrity) between the container body 21 and the cap part 22 can be identified.
(4) In the foregoing embodiments, the sealing label 1 has a rectangular shape. However, it is not particularly limited as long as it allows wrapping according to the shape of the eye drop container 2. For instance, it can be elongate circular shape.
(5) In the foregoing embodiments, the pinching portions 18a, 18b, 18c are formed integral with the sealing label 1. Alternatively, the pinching portions 18a, 18b, 18c can be formed separately from the sealing label 1, to be bonded to the sealing label 1. With this arrangement, the directions of the leading ends of the pinching portions 18a, 18b, 18c can be set to any orientation of upper/lower and left/right.
(6) The invention is not limited to the arrangement provided in the foregoing embodiments in which the cap part 22 has a truncated conical shape, and the container body 21 has an elongate circular shape. Instead, these can be any shape, for instance, both of them can be provided with a polygonal cylindrical shape.

### Industrial Applicability

The present invention is applicable to an eye drop container packaged in a sealing label.

### Reference Signs List

1: sealing label
11: upper region
12: lower region
13: cutaway region
14a, 14b, 14c: perforation line (cutaway line)
15a, 15b, 15c: perforation line (cutaway line)
2: eye drop container
21: container body
22: cap part
23: top face
3a: identifying indicium (a shape, a pattern, a color or combination thereof)
3b: identical or similar identifying indicium (a shape, a pattern, a color or combination thereof)
3c: identical or similar identifying indicium (a shape, a pattern, a color or combination thereof)

## Claims

1. An eye drop container comprising:
an identifying indicium comprising a shape, a pattern, a color or combination thereof imparted to a lateral face of a container body;
a further identifying indicium identical or similar to the identifying indicium imparted to a lateral face of a cap part which is detachably attached to a dispensing mouth of the container body, thus allowing identification of unity between the container body and the cap part; and
the container being packaged by a sealing label that renders the identifying indicium and the further identifying indicium visible when the cap part is attached to the container body.

2. An eye drop container comprising:
an identifying indicium comprising a shape, a pattern, a color or combination thereof imparted to a lateral face of a container body; and
the container being packaged by a sealing label in such a manner as to render the identifying indicium visible when a cap part that is detachably attached to a dispensing mouth of the container body is attached to the container body;
wherein a further identifying indicium identical or similar to the identifying indicium is provided in the sealing label in either an upper region for covering the cap part wholly or partially or a region comprising the upper region and a cutaway region disposed between the upper region and a lower region for covering the container body wholly or partially, thus allowing identification of unity between the container body and the cap part;
wherein in a border between the cutaway region and the upper region and in a border between the cutaway region and the lower region, a cutaway line is provided respectively; and
wherein the upper region remains attached to the cap part after unsealing of the sealing label, thus allowing identification of unity between the container body and the cap portion.

3. An eye drop container comprising:
an identifying indicium comprising a shape, a pattern, a color or combination thereof imparted to a lateral face of a cap part detachably attached to a dispensing mouth of a container body; and
the container being packaged by a sealing label that renders the identifying indicium visible when the cap part is attached to the container body;
wherein a further identifying indicium identical or similar to the identifying indicium is provided in the sealing label in a lower region thereof configured to cover the container body wholly or partially, thus allowing identification of unity between the container body and the cap part;
wherein in a border between an upper region of the sealing label configured to cover the cap part wholly or partially and the lower region, a cutaway line is provided; and
wherein the lower region remains attached to the cap part after unsealing of the sealing label, thus allowing identification of unity between the container body and the cap portion.

4. The eye drop container according to any one of claims 1-3, wherein the identifying indicium or the further identifying indicium identical or similar to the identifying indicium are provided in a plurality to the lateral face of the cap part, the lateral face of the container body or the sealing label.

5. The eye drop container according to any one of claims 1-4, wherein a further identifying indicium identical or similar to the identifying indicium is imparted to a top face of the cap part.
